# EUROPEAN PATENT APPLICATION

(11) **EP 2 392 301 A1**
(43) Date of publication of application: **07.12.2011**
(21) Application number: 10735942.4
(22) Date of filing: 01.02.2010
(51) Int. Cl.: A61F 13/15, A61F 13/472, A61F 13/534

(54) **ABSORBENT ARTICLE**

(30) Priority: 02.02.2009 JP 2009021865
(71) Applicant: Unicharm Corporation, Ehime 799-0111 (JP)
(72) Inventor: NODA, Yuki, Kanonji-shi Kagawa 769-1602 (JP); WADA, Mitsuhiro, Kanonji-shi Kagawa 769-1602 (JP); SHIRAISHI, Tsukasa, Kanonji-shi Kagawa 769-1602 (JP)
(74) Representative: Eke, Philippa Dianne
(86) International application number: PCT/JP2010/051364
(87) International publication number: WO 2010/087482

(57) **Abstract**

In an absorbent article 1, an absorber 30 is arranged between a liquid-permeable topsheet 10 and a liquid-impermeable backsheet 20, and the absorber 30 includes electrolyte particles as a hygroscopic material 70. This enables exhibition of a sufficient hygroscopicity during wearing and more surely suppresss a discomfort of a wearer due to sweatiness or tackiness.

## Description

### [Technical Field]

The present invention relates to an absorbent article including a topsheet, a backsheet, and an absorber.

### [Background Art]

Conventionally, an absorbent article such as a sanitary napkin generally includes a liquid-permeable topsheet that comes into contact with the skin of a wearer and permeates a bodily liquid such as menstrual blood discharged from the wearer, a liquid-impermeable backsheet that does not permeate the bodily liquid, and an absorber that is arranged between the topsheet and the backsheet and absorbs the bodily liquid.

In such an absorbent article, there is known a method in which a hygroscopic material using a highly water-absorptive polymer is mixed with an absorber in order to suppress a discomfort of a wearer caused due to sweatiness or tackiness during wearing (for example, Patent Document 1).

### [Prior Art Document]

### [Patent Document]

### [Patent Document 1]

Japanese Unexamined Patent Application Publication No. H06-511278 (pages 3 to 4, Fig. 1)

### [Summary of the Invention]

The aforementioned conventional absorbent article has the following problems: That is, if humidity (relative humidity) before wearing the absorbent article, specifically at the time of the storage of the absorbent article, is high, then the hygroscopic material absorbs moisture in the air, and therefore, it is not possible for the absorbent article to sufficiently absorb the moisture during wearing. In other words, there is a problem that it is not possible to demonstrate a sufficient hygroscopicity during wearing and it is not possible to sufficiently suppress a discomfort of a wearer caused due to sweatiness or tackiness during wearing.

Therefore, the present invention has been achieved in view of such a situation, and an object thereof is to provide an absorbent article that can demonstrate a sufficient hygroscopicity during wearing and can more surely suppress a discomfort of a wearer caused due to sweatiness or tackiness.

To solve the aforementioned problems, the present invention has the following characteristic. The characteristic of the present invention is an absorbent article (absorbent article 1) including a liquid-permeable topsheet (topsheet 10); a liquid-impermeable backsheet; an absorber (absorber 30) arranged between the topsheet and the backsheet (backsheet 20), in which electrolyte particles are provided as a hygroscopic material (hygroscopic material 70), and the hygroscopic material is arranged between the topsheet and the backsheet.

According to the present invention, it is possible to provide an absorbent article that can demonstrate a sufficient hygroscopicity during wearing and can more surely suppress a discomfort of a wearer caused due to sweatiness or tackiness.

### [Brief Description of Drawings]

[Fig. 1] Fig. 1 is a plan view of an absorbent article according to a first embodiment of the present invention.
[Fig. 2] Fig. 2 is a cross-sectional view taken along an F1-F1'line illustrated in Fig. 1.
[Fig. 3] Fig. 3 is an enlarged view obtained by enlarging a region A of the cross-sectional view illustrated in Fig. 2.
[Fig. 4] Fig. 4 is an enlarged cross-sectional view of an absorbent article according to a second embodiment of the present invention.
[Fig. 5] Fig. 5 is an enlarged cross-sectional view of an absorbent article according to a third embodiment of the present invention.
[Fig. 6] Fig. 6 is an enlarged cross-sectional view of an absorbent article according to a fourth embodiment of the present invention.
[Fig. 7] Fig. 7 is an enlarged cross-sectional view of an absorbent article according to a fifth embodiment of the present invention.
[Fig. 8] Fig. 8 is an enlarged cross-sectional view of an absorbent article according to a modification of the fifth embodiment of the present invention.
[Fig. 9] Fig. 9 is a graph illustrating a relationship between a moisture pick-up and a passage of time under a condition that storage is assumed.
[Fig. 10] Fig. 10 is a graph illustrating a relationship between a moisture pick-up and a passage of time under a condition that wearing is assumed.

### [Description of Embodiments]

Embodiments of an absorbent article according to the present invention will be explained with reference to the diagrams. In the following description of the diagrams, the identical or similar portions are assigned with the identical or similar numerals. However, it should be noted that the drawings are merely exemplary and ratios of each dimension differ from the actual ones.

Therefore, the specific dimensions, etc., should be determined in consideration of the following explanations. Moreover, it is needless to say that relations and ratios among the respective dimensions differ among the diagrams.

### (1) First embodiment

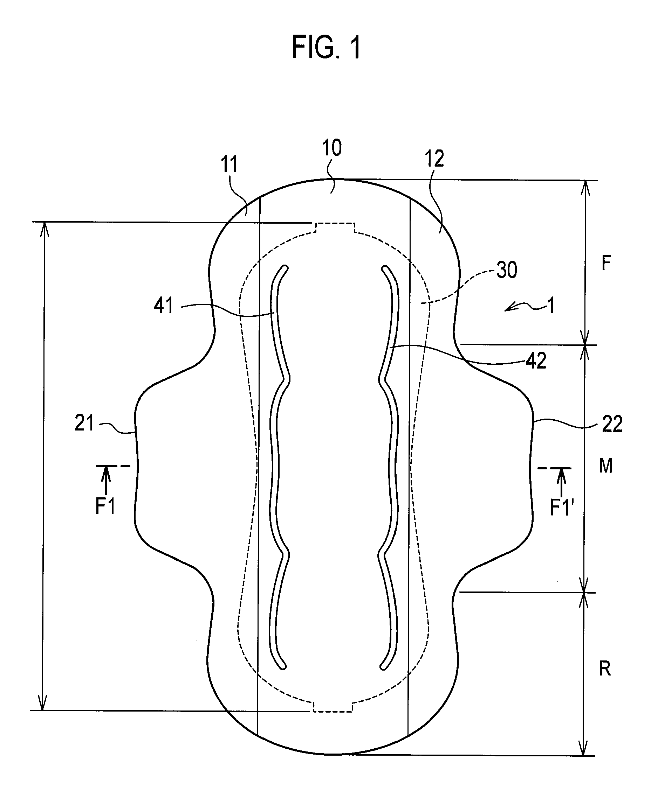

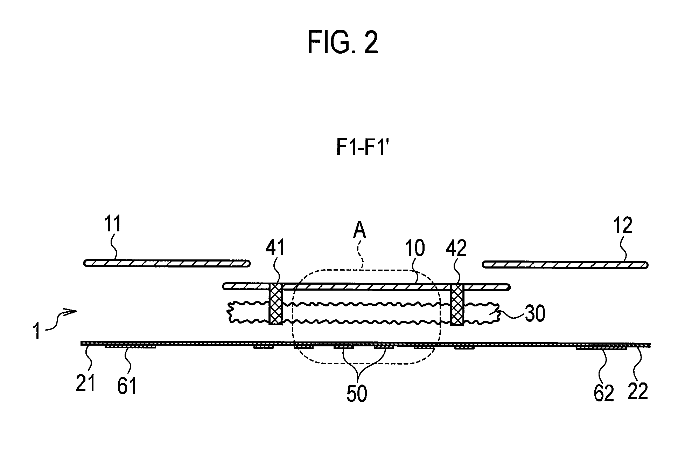

With reference to Fig. 1 and Fig. 2, an absorbent article according to a first embodiment of the present invention will be explained. Fig. 1 is a plan view of an absorbent article 1 according to the first embodiment of the present invention. The absorbent article 1 is a sanitary napkin, for example.

As illustrated in Fig. 1, the absorbent article 1 has an anterior region F, a central region M, and a posterior region R. The anterior region F contacts a skin surface at a ventral side of a wearer. The central region M contacts a skin surface around a vaginal opening of the wearer. The posterior region R contacts a skin surface at a hip side of the wearer.

The absorbent article 1 includes a liquid-permeable topsheet 10, a liquid-impermeable backsheet 20, a liquid-impermeable sidesheet 11, a sidesheet 12, and an absorber 30.

The absorber 30 is arranged between the topsheet 10 and the backsheet 20. Therefore, the absorber 30 is illustrated by a dotted line in Fig. 1. The absorber 30 is arranged at a central portion in a longitudinal direction of the absorbent article 1.

The backsheet 20 includes a wing 21 and a wing 22. The wing 21 and the wing 22 are formed as a pair at corresponding locations in a widthwise direction of the absorbent article 1. The wing 21 and the wing 22 extend in the widthwise direction of the absorbent article 1 in the central region M. The width of the central region M of the backsheet 20 is larger than the width of the anterior region F and the posterior region R.

The topsheet 10 has a length approximately identical to the length of the backsheet 20. Ends of the longitudinal directions of the topsheet 10 are approximately parallel to each other. The shape of the end of the topsheet 10 is approximately identical to the shape of the backsheet 20. The topsheet 10 covers the surface of the absorber 30.

The sidesheet 11 and the sidesheet 12 are arranged at both flanks of the topsheet 10. The sidesheet 11 covers one portion of the end of the absorber 30 and the wing 21. One end of the longitudinal direction of the sidesheet 11 is an approximate straight line, and overlaps with one end of the longitudinal direction of the topsheet 10. The other end of the longitudinal direction of the sidesheet 11 matches with the shapes of one portion of the outer circumference of the backsheet 20 and the wing 21.

The sidesheet 12 covers one portion of the side edge of the absorber 30 and the wing 22. One end of the longitudinal direction of the sidesheet 12 is an approximate straight line, and overlaps with the other end of the longitudinal direction of the topsheet 10. The other end of the longitudinal direction of the sidesheet 12 matches with the shapes of one portion of the outer circumference of the backsheet 20 and the wing 22.

Fig. 2 is a cross-sectional view taken along an F1-F1'line of the absorbent article 1. As illustrated in Fig. 2, the absorbent article 1 includes the topsheet 10, the backsheet 20, and the absorber 30. The absorber 30 is arranged between the topsheet 10 and the backsheet 20. The wing 21 is covered with the sidesheet 11. The wing 22 is covered with the sidesheet 12.

In the absorbent article 1, the topsheet 10, the sidesheet 11, the sidesheet 12, the backsheet 20, and the absorber 30 are joined to one another. The circumferential edges of the topsheet 10, the sidesheet 11, the sidesheet 12, and the backsheet 20 are joined so that the absorber 30 is contained inside. As a method of joining the topsheet 10 and the backsheet 20, any one of or a combination of two or more of a heat emboss processing, ultrasonic wave, and a hot melt-type adhesive may be selected. The topsheet 10 and the absorber 30 are pressure-bonded to each other by a pressure-bonding unit 41 and a pressure-bonding unit 42. The pressure-bonding unit 41 and the pressure-bonding unit 42 are formed along the longitudinal direction of the absorber 30 at both sides of the widthwise direction of the absorber 30. In this embodiment, the pressure-bonding unit 41 and the pressure-bonding unit 42 are pressure-bonded by way of heat emboss proce ssing.

In the backsheet 20, an adhesive member 50 is applied to a surface that comes into contact with shorts in a line shape along the longitudinal direction of the backsheet 20. A plurality of lines of adhesive members 50 are applied along the longitudinal direction of the backsheet 20. In the wing 21 and the wing 22, the adhesive member 61 and the adhesive member 62 are applied to the surface that comes into contact with the shorts.

The adhesive member 50 is bonded to a skin side of the shorts. When the wing 21 and the wing 22 are folded back, the surface that comes into contact with the shorts and the outside of the shorts come into contact. The adhesive member 61 and the adhesive member 62 are bonded to the outside of the shorts. As a result, the absorbent article 1 is fixed to the shorts.

It is noted that although not illustrated, a protective sheet to keep the adherence is boned to the adhesive member 50, the adhesive member 61, and the adhesive member 62. The protective sheet is peeled off by the wearer when in use.

The dimension of the longitudinal direction of the absorbent article 1 preferably is in the range of 100 mm to 500 mm, and specifically more preferably is in the range of 150 mm to 350 mm. Further, the dimension of the widthwise direction preferably is in the range of 30 mm to 200 mm, and specifically more preferably is in the range of 40 mm to 180 mm.

In this embodiment, the topsheet 10 is nonwoven fabric. The topsheet 10 is not particularly limited in material as long as the material has a sheet-shaped liquid-permeable structure, such as woven fabric and perforated plastic sheet. Either natural fibers or chemical fibers can be used as the woven fabric or nonwoven fabric material. Examples of the natural fibers include cellulose such as ground pulp and cotton. Examples of the chemical fibers include regenerated cellulose such as rayon and fibril rayon; semi-synthetic cellulose such as acetate and triacetate; thermoplastic hydrophobic chemical fibers; and thermoplastic hydrophobic chemical fibers that have been subjected to hydrophilic treatment. It is noted that examples of the thermoplastic hydrophobic chemical fibers include mono filament such as polyethylene (PE), polypropylene (PP), and polyethylene terephthalate (PET); fibers obtained by graft polymerization of polyethylene and polypropylene; and composite fiber with core-clad structures.

A web forming method of nonwoven fabric may include either one of a dry type (a card method, a spun bond method, a melt blown method, an air-laid method, etc.) or a wet type, or a combination of a plurality of methods. Examples of a bonding method include a thermal bonding method, a needle punch method, and a chemical bonding method; however, the present invention is not limited to the above-described methods. A spunlace formed in a sheet shape according to a hydroentangling method can be applied to the topsheet 10.

Further, in this embodiment, as the backsheet 20, a film formed mainly of polyethylene and polypropylene, a breathable resin film, or a sheet obtained by joining a breathable resin film to nonwoven fabric such as spunbond or spunlace, may be used, for example. It is preferable that the backsheet 20 is made from a material having a particular flexibility not causing a discomfort during wearing. For example, it is preferable to use a film made mainly from low-density polyethylene (LDPE) resin and having a weight (weight per unit area (g)) in a range of 15 g/m2 to 30 g/m2.

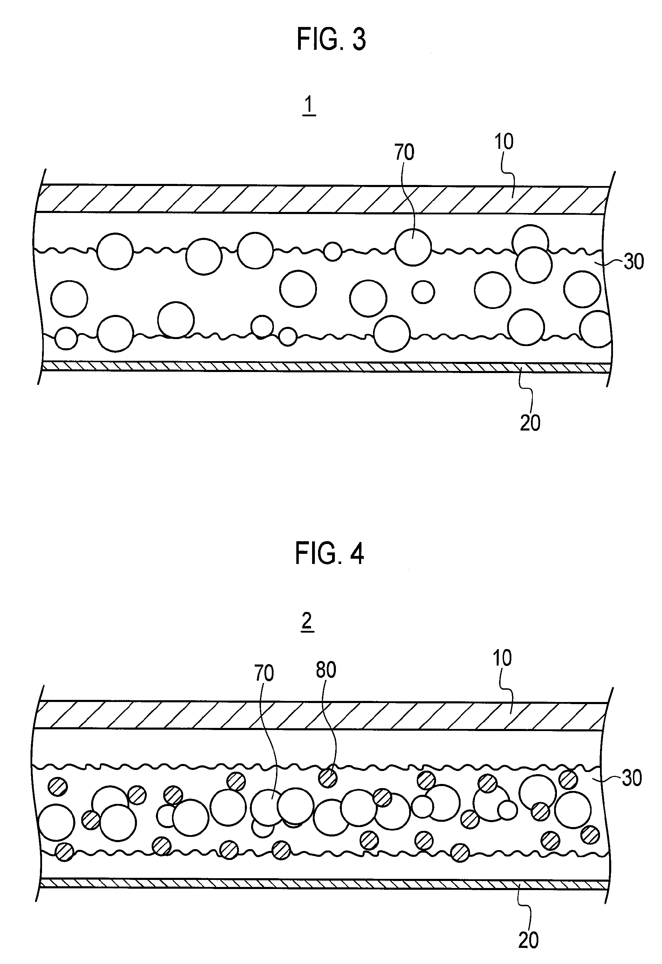

In this embodiment, the absorber 30 has electrolyte particles as a hygroscopic material 70. Fig. 3 is an enlarged view obtained by enlarging a region A of the cross-sectional view illustrated in Fig. 2. A circle in Fig. 3 is the hygroscopic material 70. In the first embodiment, the absorber 30 and the hygroscopic material 70 are arranged between the topsheet 10 and the backsheet 20.

The absorber 30 includes hydrophilic fiber and pulp. Examples of the hydrophilic fibers include cellulose such as ground pulp and cotton; regenerated cellulose such as rayon and fibril rayon; semi-synthetic cellulose such as acetate and triacetate; particulate polymers; fibrous polymers; thermoplastic hydrophobic chemical fibers; or thermoplastic hydrophobic chemical fibers that have been subjected to hydrophilic treatment. These can be used singly or intermixed. Taking into consideration low cost and ease of making into an absorber, it is preferable to use the ground pulp.

The absorber 30 may be an air-laid sheet obtained by forming hydrophilic fiber or powders into a sheet shape according to an air-laid method. When the air-laid sheet is used for the absorber 30, the thickness of the air-laid sheet preferably is from 0.3 mm to 5.0 mm. Examples of the air-laid sheet include that obtained by forming fiber and particulate polymer into a sheet article by binder. It is noted that in the air-laid sheet, the particulate polymer may be dispersed in a layered form or offset in a thickness direction.

The absorber 30 may be imparted with embossing in order to prevent losing shape and twisting during wearing, or adjust the thickness. The embossing to the absorber 30 can be formed by inserting an absorber between a patterned embossing roll and a flat roll. Examples of the pattern of the embossing roll include a lattice, a dot, and a waveform; however, a lattice pattern enabling easy thickness adjustment is preferable.

In this embodiment, the dimension of the longitudinal direction of the absorber 30 preferably is in the range of 90 mm to 490 mm. Specifically the range of 140 mm to 340 mm is more preferable. The dimension of the widthwise direction preferably is in the range of 25 mm to 100 mm. Specifically the dimension is more preferably in the range of 35 mm to 80 mm.

The hygroscopic material 70 is a material having a property in which the moisture pick-up at a temperature at the time of wearing by a wearer is larger than the moisture pick-up at a temperature before use. The hygroscopic material 70 is a material exhibiting an endoergic reaction due to hydration. Examples thereof include kalium chloride, sodium chloride, sodium acetate, and potassium nitrate. The kalium chloride is preferable from a perspective of a low irritation toward a skin when solving or a stability during a long-period storage.

The mixture amount of the hygroscopic material 70 depends on the types of materials used as a hygroscopic material; however, it is preferable to be in a range of 0.1 g to 10 g. It is more preferable to be in a range of 0.5 g to 5 g. When 0.1 g or less, it is not possible to obtain a sufficient hygroscopic effect. When exceeding 10 g, it may impart a foreign-body feeling to the wearer, and hence not preferable.

When the hygroscopic material is mixed with a polymer absorber, the mixture ratio is not limited; however, a ratio of hygroscopic material to polymer absorber = 90: 10 to 10: 90 is preferable. When a hygroscopic material having an endoergic reaction is used, it is preferable that hygroscopic material: polymer absorber = 80: 20 to 40: 60.

According to the absorbent article 1 based on the first embodiment, electrolyte particles are provided as the hygroscopic material 70 between the liquid-permeable topsheet 10 and the liquid-impermeable backsheet 20. The hygroscopic material 70 is deliquesced, while being worn, due to menstrual blood or humidity such as perspiration by the wearer, for example. In the hygroscopic material 70, once deliquescence starts, an action of absorbing surrounding moisture is promoted. Thus, the absorbent article 1, while being worn, exhibits a sufficient hygroscopicity Therefore, the absorbent article 1 can more surely suppress discomfort of a wearer caused due to sweatiness or tackiness.

In the electrolyte used as the hygroscopic material 70, the moisture pick-up at a temperature at the time of wearing by the wearer is larger than the moisture pick-up at a temperature before the use. That is, when the electrolyte particles are used as the hygroscopic material 70, the hygroscopicity is exhibited during wearing. Therefore, it is possible to prevent the moisture pick-up from losing in a pre-use state (storage state).

### (2) Second embodiment

Subsequently an absorbent article 2 according to a second embodiment of the present invention will be explained. The second embodiment has a configuration similar to that of the absorbent article 1 except that the location where the absorber 30 and the hygroscopic material 70 are arranged between the topsheet 10 and the backsheet 20 differs. Therefore, an enlarged view obtained by enlarging a region A of the cross-sectional view illustrated in Fig. 2 is used for description. As illustrated in Fig. 4, in the absorbent article 2, the absorber 30 includes a polymer absorber 80. The hygroscopic material 70 is arranged inside the absorber 30. A hatched circle in Fig. 4 indicates the polymer absorber 80. The hygroscopic material 70 and the polymer absorber 80 are both included in the absorber 30. As the polymer absorber 80, a granular polymer such as absorbent or hygroscopic sodium arylate copolymer may be generally used.

The hygroscopic material 70 is deliquesced into an aqueous solution. In the absorbent article 2, the absorber 30 includes the polymer absorber 80, and the hygroscopic material 70 is arranged inside the absorber 30. Thus, it is possible to absorb the aqueous solution obtained as a result of deliquesce of the hygroscopic material 70, by the polymer absorber 80. Therefore, the absorbent article 2 can more surely suppress a discomfort of a wearer caused due to sweatiness or tackiness.

When the hygroscopic material 70 is used together with the polymer absorber 80, it is preferable that the particle diameter of the hygroscopic material 70 mainly is 500 µm or less, and it is preferable that the particle diameter of the polymer absorber 80 mainly is 400 µm or less. When the particle diameter size of the hygroscopic material 70 and the particle diameter size of the polymer absorber 80 are close and close to the above-described numeral values, a capability of handling the hygroscopic material 70 and the polymer absorber 80 can be improved in terms of manufacture.

### (3) Third embodiment

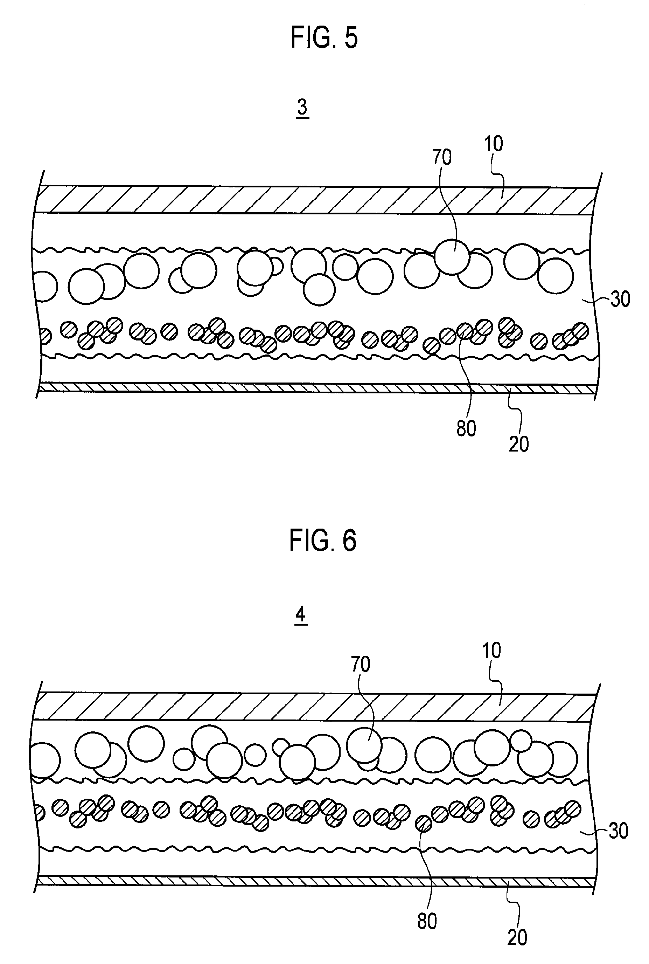

Subsequently an absorbent article 3 according to a third embodiment of the present invention will be explained. The third embodiment has a configuration similar to that of the absorbent article 1 except that a location where the absorber 30 and the hygroscopic material 70 are arranged between the topsheet 10 and the backsheet 20 differs. Therefore, an enlarged view obtained by enlarging a region A of the cross-sectional view illustrated in Fig. 2 is used for description. As illustrated in Fig. 5, in the absorbent article 3, the absorber 30 includes a polymer absorber 80. The hygroscopic material 70 is arranged inside the absorber 30. Ahatched circle in Fig. 5 indicates the polymer absorber 80. In the third embodiment, the hygroscopic material 70 and the polymer absorber 80 are both included in the absorber 30. The hygroscopic material 70 is arranged closer to the topsheet 10 than the polymer absorber 80.

The hygroscopic material 70 is deliquesced into an aqueous solution. In the absorbent article 3, the absorber 30 includes the polymer absorber 80, and the hygroscopic material 70 is arranged inside the absorber 30. Further, the hygroscopic material 70 is arranged closer to the topsheet 10 than the polymer absorber 80. That is, the hygroscopic material 70 is arranged closer to the skin of the wearer. This increases the temperature of the hygroscopic material 70, making it easier to exhibit a hygroscopicity Moreover, it is possible to efficiently absorb the humidity retained near space close to the skin.

In the thickness direction of the absorbent article 3, the hygroscopic material 70 is arranged above the polymer absorber 80. As a result, when the aqueous solution obtained by deliquescence of the hygroscopic material 70 descends due to the influence of gravity it is possible to efficiently absorb the solution by the polymer absorber 80. Therefore, the absorbent article 2 can more surely suppress a discomfort of a wearer caused due to sweatiness or tackiness.

Similarly to the absorbent article 2, when the hygroscopic material 70 is used together with the polymer absorber 80, it is preferable that the particle diameter of the hygroscopic material 70 mainly is 500 µm or less, and it is preferable that the particle diameter of the polymer absorber 80 mainly is 400 µm or less. There is an advantage that when the particle diameter of the hygroscopic material 70 arranged above the polymer absorber 80 is larger than the particle diameter of the polymer absorber 80, it is easy to absorb the humidity and moisture at an upper layer side of the absorbent article 3.

### (4) Fourth embodiment

Subsequently an absorbent article 4 according to a fourth embodiment of the present invention will be explained. The fourth embodiment has a configuration similar to that of the absorbent article 1 except that a location where the absorber 30 and the hygroscopic material 70 are arranged between the topsheet 10 and the backsheet 20 differs. Therefore, an enlarged view obtained by enlarging a region A of the cross-sectional view illustrated in Fig. 2 is used for description. As illustrated in Fig. 6, in the absorbent article 4, the absorber 30 includes a polymer absorber 80. The hygroscopic material 70 is arranged closer to the topsheet 10 than the absorber 30.

In the absorbent article 4, the hygroscopic material 70 is arranged closer to the topsheet 10 than the absorber 30, resulting in a promoted deliquescence. This serves to further facilitate the provision of a hygroscopicity. Moreover, it is possible to efficiently absorb the humidity retained near space close to the skin. When the aqueous solution obtained by deliquescence of the hygroscopic material 70 descends due to the influence of gravity it is possible to efficiently absorb the solution by the absorber 30 and the polymer absorber 80. Therefore, the absorbent article 2 can more surely suppress a discomfort of a wearer caused due to sweatiness or tackiness.

It is preferable that the particle diameter of the hygroscopic material 70 mainly is 500 µm or less, and it is preferable that the particle diameter of the polymer absorber 80 mainly is 400 µm or less. There is an advantage that when the particle diameter of the hygroscopic material 70 arranged above the polymer absorber 80 is larger than the particle diameter of the polymer absorber 80, it is easy to absorb the humidity and moisture at an upper layer side of the absorbent article 4.

### (5) Fifth embodiment

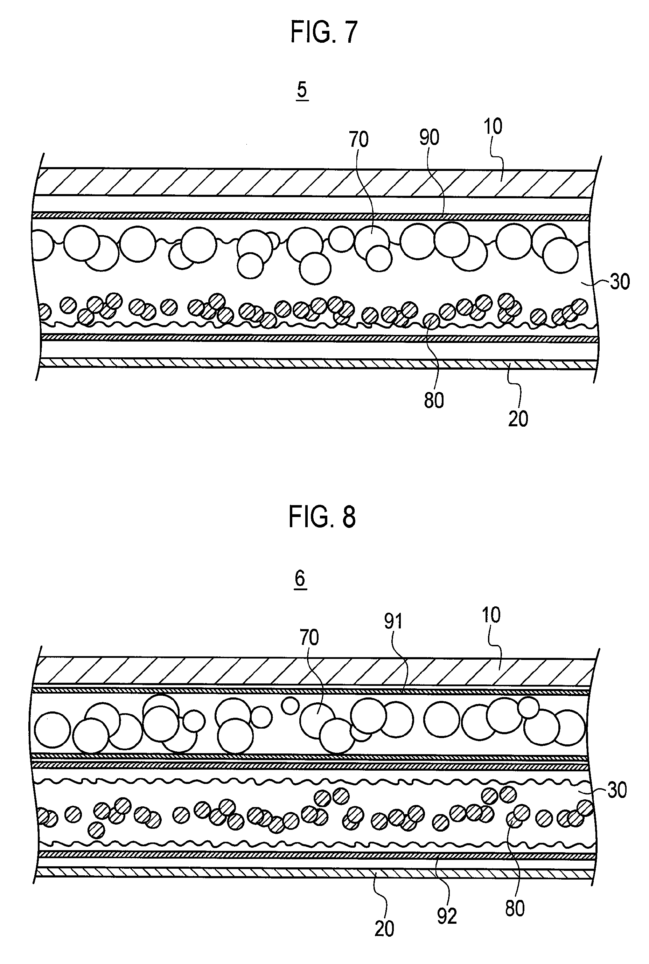

Subsequently an absorbent article 5 according to a fifth embodiment of the present invention will be explained. The fifth embodiment has a configuration similar to that of the absorbent article 1 except that a location where the absorber 30 and the hygroscopic material 70 are arranged between the topsheet 10 and the backsheet 20 differs. Therefore, an enlarged view obtained by enlarging a region A of the cross-sectional view illustrated in Fig. 2 is used for description. As illustrated in Fig. 7, the absorbent article 5 includes a hydrophilic sheet 90 including hydrophilic fiber having a high affinity relative to water. In the absorbent article 5, the absorber 30, the hygroscopic material 70, and the polymer absorber 80 are covered with the hydrophilic sheet 90. The hygroscopic material 70 comes into contact with the hydrophilic sheet 90. Examples of the hydrophilic sheet 90 include a tissue.

The hydrophilic sheet 90 can hold moisture such as menstrual blood or perspiration of the wearer. The moisture spreads over the hydrophilic sheet 90. The hygroscopic material 70 comes into contact with the hydrophilic sheet 90. Thus, it is possible to disperse a small amount of moisture over the hydrophilic sheet 90, and it is possible to cause the hygroscopic material 70 present in a wider range to absorb the moisture. This promotes the deliquesce of the hygroscopic material 70. As a result, it is possible for the hygroscopic material 70 to exhibit the hygroscopicity

Moreover, it is possible to efficiently absorb the humidity retained near space close to the skin because the hygroscopic material 70 is arranged near the skin. In the thickness direction of the absorbent article 5, the hygroscopic material 70 is arranged above the polymer absorber 80. As a result, the aqueous solution obtained as a result of deliquesce of the hygroscopic material 70 descends due to the influence of gravity and effectively absorbed by the polymer absorber 80. Therefore, the absorbent article 5 can more surely suppress a discomfort of a wearer caused due to sweatiness or tackiness.

It is preferable that the particle diameter of the hygroscopic material 70 mainly is 500 µm or less, and it is preferable that the particle diameter of the polymer absorber 80 mainly is 400 µm or less. There is an advantage that when the particle diameter of the hygroscopic material 70 arranged above the polymer absorber 80 is larger than the particle diameter of the polymer absorber 80, it is easy to absorb the humidity and moisture at an upper layer side of the absorbent article 5.

An absorbent article 6 illustrated as a modification of the fifth embodiment will be explained. As illustrated in Fig. 8, the absorbent article 6 includes a hydrophilic sheet 91 and a hydrophilic sheet 92 including hydrophilic fiber. That is, the absorber 30 and the hygroscopic material 70 are covered with respectively different hydrophilic sheets. In the absorbent article 6, the hygroscopic material 70 is covered with the hydrophilic sheet 91. The hygroscopic material 70 comes into contact with the hydrophilic sheet 91. In the hydrophilic sheet 91, the hygroscopic material 70 and the absorber 30 may be included.

The absorber 30 and the polymer absorber 80 are covered with the hydrophilic sheet 92. The hygroscopic material 70 comes into contact with the hydrophilic sheet 90. Example of the hydrophilic sheet 91 and the hydrophilic sheet 92 include a tissue.

Thus, the content of the present invention is disclosed through the first to fifth embodiments of the present invention. However, it should not be interpreted that the statements and drawings constituting a part of the present disclosure limit the present invention. From this disclosure, a variety of alternate embodiments, examples, and applicable techniques will be apparent to one skilled in the art.

For example, the embodiment of the present invention can be modified as follows. In each of the above-described embodiments, the absorbent article is described as a sanitary napkin; however, the present invention may also be applied to a so-called liner, an incontinent article (called an incontinence pad), etc.

The absorbent article is not limited to a plan shape disclosed in the preceding Fig. 1. The shape of a crotch of a wearer, or any shape that adapts to the shape of shorts may be selected. The plan shape of the absorbent article may be selected from a rectangle, an ellipse, and a gourd, for example.

The absorbent article may be configured such that gathers formed by using an elastic member, for example, are arranged at the both width-direction ends of the absorber in order to prevent a side leakage of a bodily liquid such as menstrual blood.

As the polymer absorber, a granular polymer such as absorbent or hygroscopic sodium acrylate copolymer may be generally used. Between the topsheet and the backsheet, in addition to the absorber, the polymer absorber, and the hygroscopic material, granular deodorant such as silver, copper, zinc, silica, activated carbon, an aluminosilicate compound, and zeolite may be arranged.

Certain granular deodorant such as silver, copper, zinc, silica, activated carbon, an aluminosilicate compound, zeolite, and electrolyte that can be used as a hygroscopic material is used may exhibit an effect of suppressing the breeding of bacteria (antibacterial effect or bactericidal effect). For example, when the electrolyte having an effect of suppressing the breeding of bacteria is used as a hygroscopic material, it is possible to impart the absorbent article with an effect of suppressing the breeding of bacteria.

Thus, needless to say the present invention includes various embodiments not described herein. Therefore, the technical range of the present invention is to be defined only by the inventive specific matter according to the adequate claims from the above description.

### [Example]

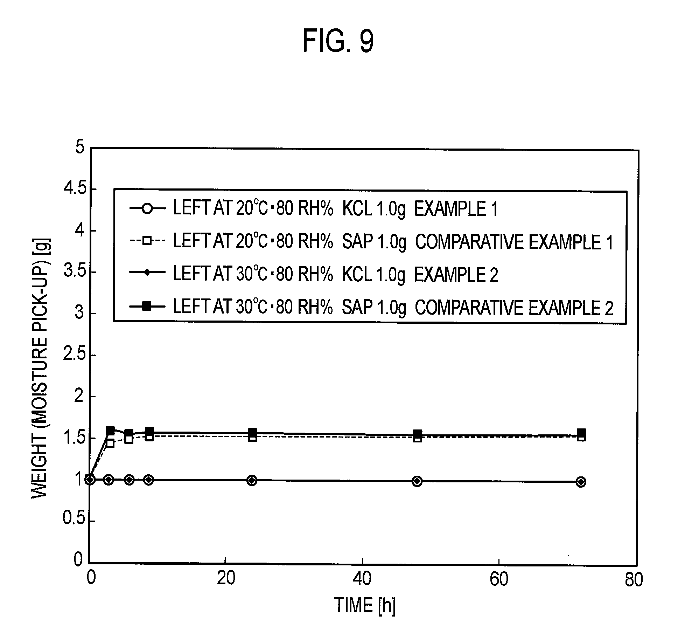

Absorbent articles (examples 1 and 2) in which between a liquid-permeable topsheet and a liquid-impermeable backsheet, 1.0 g of kalium chloride was arranged as a hygroscopic material were prepared and left for 72 hours under a condition that storage was assumed. As comparative examples, absorbent articles (comparative examples 1 and 2) in which 1.0 g of polymer absorber was arranged between a liquid-permeable topsheet and a liquid-impermeable backsheet were prepared and left under the same condition. The results are shown in Fig. 9.

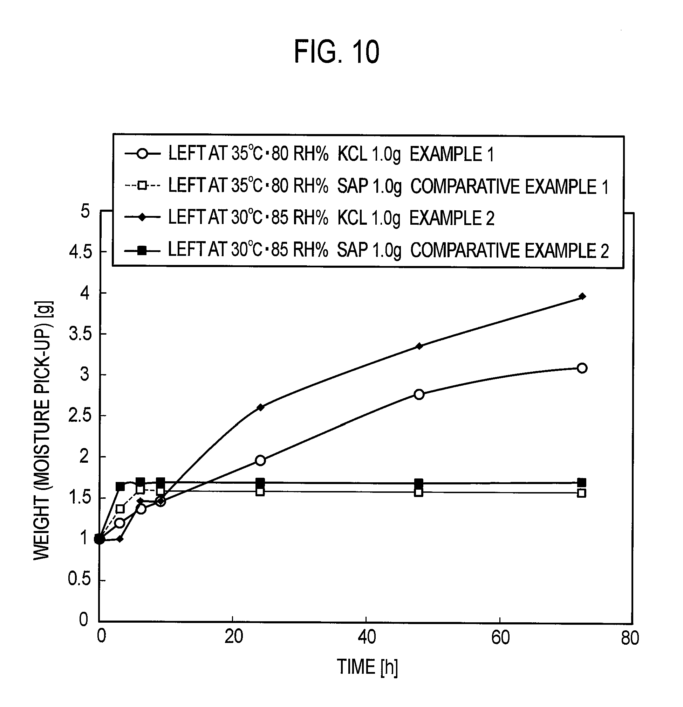

It is noted that, a condition 1 under which storage was assumed: temperature 20(C/humidity 80 RH%; and a condition 2 under which storage was assumed: temperature 30(C/humidity 80 RH%
The absorbent articles of the examples 1 and 2 were left for 72 hours under a condition that wearing was assumed. The absorbent articles of the comparative examples 1 and 2 were left under the same condition. The results are shown in Fig. 10.

It is noted that, a condition 3 under which wearing was assumed: temperature 35(C/humidity 80 RH%; and a condition 4 under which wearing was assumed: temperature 35(C/humidity 85 RH%.
According to the results, the absorbent articles based on the examples 1 and 2 in which kalium chloride was used as the hygroscopic material hardly absorbed a moisture even if time elapsed under the storage conditions 1 and 2. In contrary the absorbent articles based on the comparative examples 1 and 2 absorbed a moisture even under the storage conditions 1 and 2. Therefore, it was found that the absorbent articles based on the comparative examples 1 and 2 exhibited a deteriorated hygroscopicity during wearing by as much as the moisture pick-up during storage.

Further, the absorbent articles based on the examples 1 and 2 hardly absorbed a moisture during storage; however, under the conditions 3 and 4 that wearing was assumed, exhibited an increased moisture pick-up. That is, the moisture pick-up of the kalium chloride increased in an environment which was hot and humid. The absorbent articles based on the examples 1 and 2 hardly absorbed a moisture during storage, and therefore, there was no hygroscopicity loss during storage, and the hygroscopicity increased during wearing.

It is noted that the entire contents of Japanese Patent Application No. 2009-021865 (filed on February 2, 2009) are hereby incorporated in the present specification by reference.

### [Industrial Applicability]

According to the present invention, it is possible to exhibit a sufficient hygroscopicity during wearing and it is possible to more surely suppress a discomfort of a wearer caused due to sweatiness or tackiness, and therefore, it is possible to adapt the present invention to an absorbent article such as a liner and an incontinence pad).

### [Explanation of Numerals]

1,2,3,4,5,6: absorbent article, 10: topsheet, 11: sidesheet, 11: sidesheet, 12: sidesheet, 20: backsheet, 21: wing, 22: wing, 30: absorber, 41: pressure-bonding unit, 42: pressure-bonding unit, 50: adhesive member, 61: adhesive member, 62: adhesive member, 70: hygroscopic material, 80: polymer absorber, 90: hydrophilic sheet, 91: hydrophilic sheet, 92: hydrophilic sheet, A: region, F: anterior region, M: central region, R: posterior region

## Claims

1. An absorbent article, comprising:
a liquid-permeable topsheet;
a liquid-impermeable backsheet; and
an absorber arranged between the topsheet and the backsheet, wherein
electrolyte particles are provided as a hygroscopic material, and
the hygroscopic material is arranged between the topsheet and the backsheet.

2. The absorbent article according to claim 1, wherein
the absorber includes a polymer absorber, and the hygroscopic material is arranged inside the absorber.

3. The absorbent article according to claim 2, wherein
the hygroscopic material is arranged closer to a topsheet side than the polymer absorber.

4. The absorbent article according to claim 2 or 3, wherein the absorbent article comprises a hydrophilic sheet including hydrophilic fiber, the absorber is enveloped by the hydrophilic sheet, and the hygroscopic material comes into contact with the hydrophilic sheet.

5. The absorbent article according to any one of claims 1 to 4, wherein the hygroscopic material is kalium chloride.

6. The absorbent article according to any one of claims 1 to 5, wherein a moisture pick-up of the hygroscopic material at a temperature during wearing is larger than a moisture pick-up at a temperature before use.

7. The absorbent article according to claim 1, wherein the absorber includes the polymer absorber, and the hygroscopic material is arranged closer to a topsheet side than the absorber.
